# EUROPEAN PATENT SPECIFICATION

(11) **EP 3 024 546 B1**
(45) Date of publication and mention of the grant of the patent: **18.12.2019**
(21) Application number: 14752667.7
(22) Date of filing: 08.07.2014
(51) Int. Cl.: A61N 5/06

(54) **SYSTEM FOR PROVIDING LIGHT THERAPY AND MODIFYING CIRCADIAN RHYTHM**
SYSTEM ZUR VERABREICHUNG EINER LICHTTHERAPIE UND ZUM VERÄNDERN DES ZIRKADIANEN RHYTHMUS
SYSTÈME POUR FOURNIR UNE LUMINOTHÉRAPIE ET MODIFIER LE RYTHME CIRCADIEN

(30) Priority: 25.07.2013 US 201361858163 P
(43) Date of publication of application: 01.06.2016
(73) Proprietor: Koninklijke Philips N.V., 5656 AE Eindhoven (NL)
(72) Inventor: COLBAUGH, Michael Edward, 5656 AE Eindhoven (NL); LUCCI, Christopher Scott, 5656 AE Eindhoven (NL)
(74) Representative: Schudelaro, Antonius Adrianus Petrus
(86) International application number: PCT/IB2014/062945
(87) International publication number: WO 2015/011589

(56) References cited:
- WO-A1-2009/023968
- WO-A1-2010/076709
- WO-A1-2010/122446
- WO-A1-2013/084104

## Description

### BACKGROUND

### 1. Field

The present disclosure pertains to a system and method for modifying the circadian rhythm of a subject through light therapy, and, in particular, to using pulses of electromagnetic radiation in a particular wavelength range to accomplish certain such modifications.

### 2. Description of the Related Art

The impingement of radiation on subjects to impact the circadian rhythms and/or to address light deficient disorders may be known. Generally, these treatments involve shining light directly towards a patient's eyes while the patient is awake to alleviate or cure light deficient disorders including Seasonal Affective Disorder (SAD), circadian sleep disorders and circadian disruptions associated with, *e.g.*, jet-lag, and shift-work.

There are two types of light therapy devices presently available. One type of device is large in size and floor or desk mountable. These devices include light sources of fluorescent bulbs or large arrays of light emitting diodes. Although they can be moved from one position to another, they are not generally portable and require a scheduled time period of being stationary during the active part of the day. In addition, the light source is quite fragile. The second kind of light therapy device is head mountable. These devices are formed as eyeglasses or visors. While they are portable, they are not generally accepted by patients for use in public because of their odd appearance when worn on the head. These devices generally lack features that enable them to be used while functioning during sleep. This second type of device mostly used focused or non-diffuse light sources to direct high luminance light towards the eyes.

Further, the lights are positioned to emit beams of light at the eyes of the patient while the patient is awake. This approach may impact the comfort of the treatment to the subject.

WO2009/023968 relates to an artificial light system for modulating circadian rhythms, increasing vigilance and influencing light-associated psychological conditions such as seasonal affective disorder. The system of the invention comprises a source of a green and/or red light and a source of blue light both light sources being controlled by a computer to provide predetermined light conditions. More specifically, the computer is programmed to provide pulses of blue light and continuous or pulsed red light, to enhance the efficacy of blue light, reduce blue-light hazard and avoid stroboscopic effect.

WO2010/122446 relates to a method and an illumination device arranged for reducing sleep mertia and/or for controlling alertness of a human being. The illumination device is arranged for reducing sleep inertia and/or controlling alertness by light radiation. The illumination device comprises one or more light sources for generating a first illumination output (I) in the range of 590-770 nm and a second illumination output (II) in the range of 400-560 nm. The illumination device comprises a controller configured for controlling the one or more light sources to expose said eye of said human being to the first illumination output (I) during a first time interval (Tl) and to the second illumination output (II) during a second time interval (T2). The second time interval (T2) terminates at a later point in time than the first time interval (Tl). The time intervals are selected such that at least one of the first and second time interval is less than 60 seconds.

WO2013/084104 relates to an illumination system is provided comprising a light source and a controller and being configured to provide an illumination signal for, when perceived by a mammalian, in particular human, subject, inducing relaxing in the subject. The signal comprises a plurality of light pulses having a pulse duration and being separated by inter-pulse intervals. The light pulses are grouped in stimuli which have a stimulus duration and are separated by inter-stimuli intervals. The stimuli are grouped in stimulation sequences which have a stimulation sequence duration and are separated by inter-sequence intervals. An illumination signal, a method, and a computer program product are also provided.

### SUMMARY

Accordingly, it is an object of one or more embodiments of the present invention to provide a system to provide light therapy to a subject. The system comprises one or more light sources configured to emit electromagnetic radiation at different intensities and/or having different parameters. The system further comprises one or more processors configured to execute computer program modules. The computer program modules comprise a therapy module and a light control module. The therapy module is configured to obtain a light therapy regimen for the subject. The light therapy regimen defines stages including a first stage and a second stage. The first stage is associated with a first period during one or more days. The second stage is associated with a second period during the one or more days that is different from the first period. The light therapy regimen is configured such that during the first stage the subject receives pulsed electromagnetic radiation including electromagnetic radiation having a first range of wavelengths, the electromagnetic radiation during pulses being at a first intensity, the first intensity being sufficient to stimulate S-cone receptors of the subject upon impingement. The pulsed electromagnetic radiation received during the first period has a pulse duration up to about 10 minutes and an inter-pulse duration between about 0.1 seconds and about 10 minutes. The light therapy regimen is further configured such that during the second stage the subject receives electromagnetic radiation. One or more parameters of the electromagnetic radiation during the second stage are changed from the pulsed electromagnetic radiation during the first stage such that light therapy provided during the first stage is different from light therapy provided during the second stage. The light control module is configured to control emission from the one or more light sources in accordance with the light therapy regimen.

It is yet another aspect of one or more embodiments of the present invention to provide a method to provide light therapy to a subject. The method comprises obtaining a light therapy regimen for the subject, wherein the light therapy regimen defines stages including a first stage and a second stage, wherein the first stage is associated with a first period during one or more days, wherein the second stage is associated with a second period during the one or more days that is different from the first period, wherein the light therapy regimen includes electromagnetic radiation impinging on a closed eyelid of the subject to provide light therapy for the subject; emitting, during the first stage, pulsed electromagnetic radiation including electromagnetic radiation having a first range of wavelengths, the electromagnetic radiation during pulses being at a first intensity, the first intensity being sufficient to stimulate S-cone receptors of the subject upon impingement, wherein the pulsed electromagnetic radiation emitted during the first period has a pulse duration up to about 10 minutes and an inter-pulse duration between about 0.1 seconds and about 10 minutes; and emitting, during the second stage, electromagnetic radiation, wherein one or more parameters of the electromagnetic radiation during the second stage are changed from the pulsed electromagnetic radiation during the first stage such that light therapy provided during the first stage is different from light therapy provided during the second stage.

It is yet another aspect of one or more embodiments to provide a system configured to provide light therapy to a subject. The system comprises means for obtaining a light therapy regimen for the subject, wherein the light therapy regimen defines stages including a first stage and a second stage, wherein the first stage is associated with a first period during one or more days, wherein the second stage is associated with a second period during the one or more days that is different from the first period, wherein the light therapy regimen includes electromagnetic radiation impinging on a closed eyelid of the subject to provide light therapy for the subject; means for emitting, during the first stage, pulsed electromagnetic radiation including electromagnetic radiation having a first range of wavelengths, the electromagnetic radiation during pulses being at a first intensity, the first intensity being sufficient to stimulate S-cone receptors of the subject upon impingement, wherein the pulsed electromagnetic radiation emitted during the first period has a pulse duration up to about 10 minutes and an inter-pulse duration between about 0.1 seconds and about 10 minutes; and means for emitting, during the second stage, electromagnetic radiation, wherein one or more parameters of the electromagnetic radiation during the second stage are changed from the pulsed electromagnetic radiation during the first stage such that light therapy provided during the first stage is different from light therapy provided during the second stage.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIGs. 1-3 illustrate a system configured to provide light therapy to a subject, in accordance with one or more embodiments;
FIG. 4 illustrates a system in accordance with one or more embodiments.
FIG. 5 illustrates a graph depicting transmittance of the human eyelid (on a logarithmic scale) versus wavelength.
FIGs. 6A-C illustrate variations for appliances carrying a light source.
FIG. 7 schematically illustrates the components of a system to provide light therapy according to one or more embodiments.
FIG. 8 illustrates a method of waking up a subject by emitting radiation onto a closed eyelid of the subject, according to one or more embodiments.
FIG. 9A illustrates a graph depicting required irradiance for constant melatonin suppression versus monochromatic wavelength of electromagnetic radiation.
FIG. 9B illustrates a graph depicting relative response time of retinal cone receptors versus wavelength.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, *i.e.*, through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other. As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (*i.e.*, a plurality). As used herein, the term "include" shall be used inclusively to mean any item of a list, by example and without limitation, and/or any combination of items in that list, to the extent possible.

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

Mammalian circadian systems coordinate the timing of an animal's physiological and behavioral functions with local position on the planet. The circadian system depends primarily upon the 24-hour light-dark pattern incident on the retinae. The phototransduction mechanisms responsible for human circadian phototransduction have been elucidated well enough that devices may take advantage of this understanding and adjust circadian timing as desired.

The central photo-sensor for circadian phototransduction is a class of neurons known as intrinsically photosensitive Retinal Ganglion Cells (ipRGCs). These neurons are a part of the phototransduction mechanism; the more distal rod and cone photoreceptors also participate in this process via amacrine and bipolar cells. Photic information generated by these distal photoreceptors may not be communicated directly to the intrinsically photosensitive Retinal Ganglion Cells, but may be communicated through a plexus of retinal neurons. Spectral opponent neurons underlying human color vision are also part of the circadian phototransduction mechanism. In particular, short-wavelength sensitive (S) cones may provide depolarizing input to the intrinsically photosensitive Retinal Ganglion Cells through blue versus yellow (b-y), spectral opponent bipolar cells. Due to the neurophysiology of the circadian phototransduction mechanism, responses by the b-y bipolar to wavelengths shorter than approximately 510 nm (the cross point between depolarizing and hyperpolarizing responses to monochromatic light by these b-y bipolar cells) may add to the photic response by the intrinsically photosensitive Retinal Ganglion Cells, but wavelengths longer than approximately 510 nm cannot. As described elsewhere in this disclosure, different types of electromagnetic radiation having wavelengths to which S-cone, M-cone, and/or L-cone receptors are sensitive may be combined and/or alternated to provide light therapy in general, and modify the circadian rhythm of a subject in particular.

Compared to the distal photoreceptors in the retina, direct light stimulation of the intrinsically photosensitive Retinal Ganglion Cells may exhibit a high threshold and when stimulated may be slow to respond. Once stimulated, intrinsically photosensitive Retinal Ganglion Cells may exhibit a persistent response to light well after the light has been extinguished. For example, S-cones, acting through the b-y bipolar, may respond to relatively low intensity, brief pulses of light that would not necessarily stimulate the intrinsically photosensitive Retinal Ganglion Cells. Since S-cones participate in circadian phototransduction, a train of brief pulses of short-wavelength light (< 510 nm) may be able to activate and/or stimulate the circadian system directly and/or through persistent, transient stimulation of the intrinsically photosensitive Retinal Ganglion Cells. In the latter case, because of the inherent persistence of response by the intrinsically photosensitive Retinal Ganglion Cells, intermittent stimulation of the intrinsically photosensitive Retinal Ganglion Cells from S-cone input may provide prolonged responses by these neurons.

In some embodiments, for some types and/or stages of light therapy, the emitted electromagnetic radiation may stimulate the intrinsically photosensitive Retinal Ganglion Cells and/or the S-cone receptors. The intrinsically photosensitive Retinal Ganglion Cells are a type of nerve cell in the retina. The intrinsically photosensitive Retinal Ganglion Cells may be non-image-forming, and/or may provide a stable representation of ambient light intensity. As a result, the intrinsically photosensitive Retinal Ganglion Cells may participate, without limitation, in at least three areas: (1) they may play a role in synchronizing circadian rhythms to the light/dark cycle by providing length of day, length of night, and night-to-day and day-to-night transitional information, (2) they may contribute to the regulation of pupil size, and (3) they may contribute to photic regulation of, and acute photic suppression of, release of the hormone melatonin. By virtue of the sensitivity profile of the intrinsically photosensitive Retinal Ganglion Cells for electromagnetic radiation in the visible spectrum, conventional light therapy systems, in particular those intended for open eyes, have focused on the wavelength band around 480 nm, and may utilize continuous illumination, targeting direct stimulation of the intrinsically photosensitive Retinal Ganglion Cells (preferably at peak sensitivity). For convenience, within this disclosure, the term "sleep cycle" will be used to refer to the circadian rhythms, and/or the production, suppression, and/or release of melatonin for the subject.

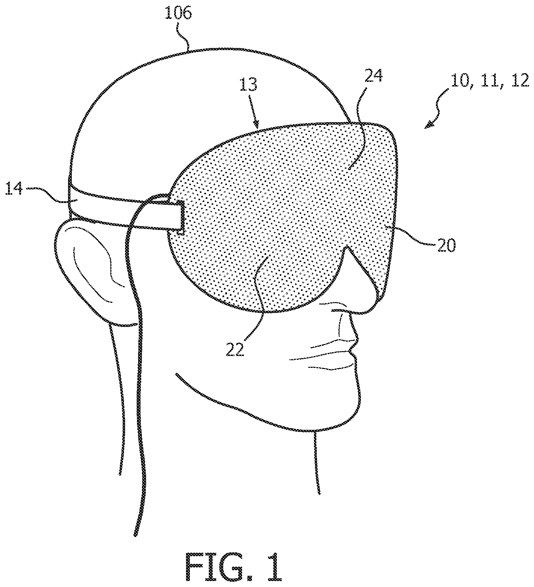

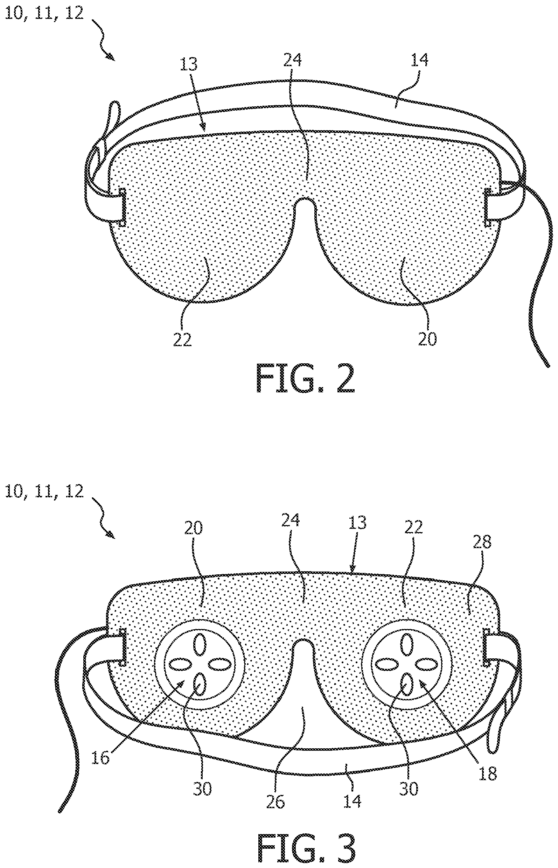

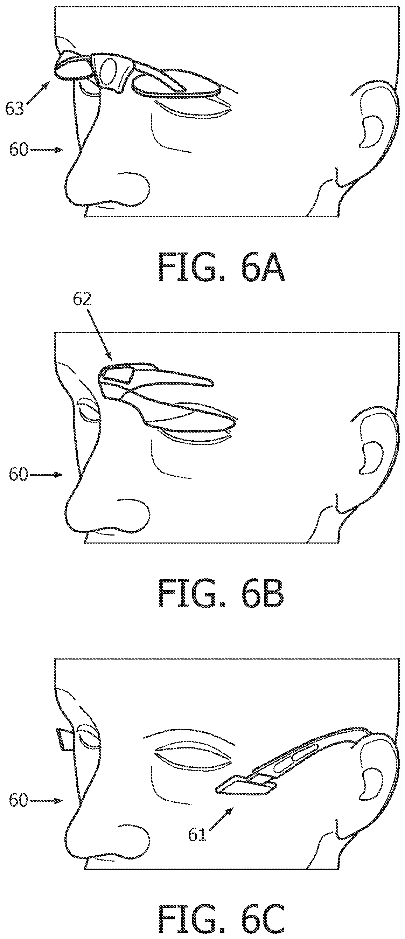

FIGs. 1-3 illustrate a system 10 configured to provide light therapy to a subject 106, in accordance with one or more embodiments. System 10 may be implemented as a sleep mask 12 (as depicted in FIG. 1), an appliance 11 configured to be worn near and/or in an eye of subject 106 and/or to carry a light source in suitable proximity to subject 106, and/or implemented in other ways/devices. The implementation using sleep mask 12 is not intended to be limiting in any way. For example, additional implementations are described in relation to FIG. 6A-C. Returning to FIGs. 1-3, note that FIGs. 1-2 depict the side of system 10 facing away from subject 106, whereas FIG. 3 depicts the side of system 10 facing towards subject 106.

System 10 may be used to treat, alleviate, and/or cure light deficient disorders including Seasonal Affective Disorder (SAD), Delayed Sleep Phase Syndrome (DSPS), Advanced Sleep Phase Syndrome (ASPS), circadian sleep disorders, and/or circadian disruptions associated with, e.g., jet-lag and/or shift-work.

System 10 is configured to provide light therapy by emitting electromagnetic radiation in such a way that the electromagnetic radiation impinges on subject 106, and, in particular, on one or more eyes and/or eyelids of subject 106. For the purposes of this disclosure, the term "eyelid" may be considered part of the subject's "eye" such that electromagnetic radiation impinging on an eye includes impingement on an eyelid, and/or *vice versa.* Emission of electromagnetic radiation in system 10 may be responsive to a determination that one or both eyes of subject 106 are closed and/or a determination that subject 106 is asleep or in a particular sleep stage. In embodiments wherein system 10 is used to modify a characteristic of the circadian rhythm of subject 106, including but not limited to the phase of the circadian rhythm, system 10 may be configured such that the emitted electromagnetic radiation impinges on one or more closed eyelids of subject 106. Common approaches to modification of the circadian rhythm include systems that provide light therapy while the subject is awake, though these approaches suffer from various practical problems, including the low level of comfort, ease, and usability for a subject.

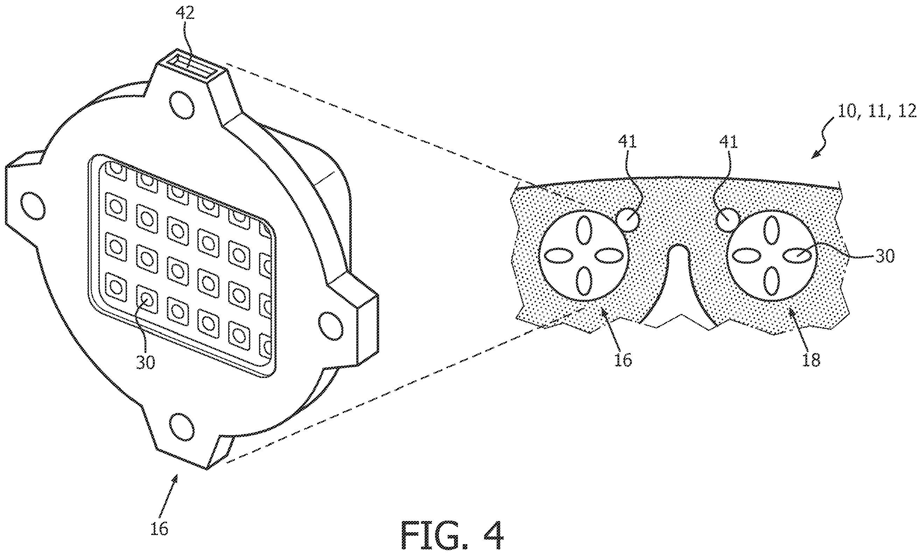

System 10 may include one or more of one or more light sources 30, a sleep mask 12, an appliance 11, a shield 13, a strap 14, a first lighting module 16, a second lighting module 18, a first shield portion 20, a second shield portion 22, a connecting shield portion 24, a cushioning layer 28, an impermeable base surface 26, an eyelid detector 41 (not shown in FIGs. 1-3, but depicted in FIG. 4), and/or other components.

In some embodiments, system 10 is implemented as appliance 11. Appliance 11 may be configured to be worn near and/or in an eye of the subject. As used herein, near the eye means within 20 cm of the eye, within 3 inches of the eye, within one inch of the eye, within a range of 1 cm to 6 cm of the eye, within a range of 0.5 to 1.0 inch of some particular part of the eye, within 0.25 inch of the cornea, contacting the eyebrow of the subject, and/or contacting the eye and/or eyelid of the subject. Appliance 11 is configured to carry one or more light sources 30 and/or one or more constituent components of system 10 that are configured to include one or more light sources 30. For example, in certain embodiments, one or more lighting modules - such as lighting module 16 and/or lighting module 18 - may each include one or more light sources 30. Note that the depiction of four light sources 30 per lighting module in FIG. 3 is exemplary and not intended to be limiting in any way. In some embodiments, appliance 11 may include a contact lens. In some embodiments, appliance 11 may include a lamp fixture that is standing or mounted above the subject.

One or more light sources 30 are configured to emit electromagnetic radiation such that the emitted electromagnetic radiation impinges on one or more eyes and/or eyelids of subject 106. Light source 30 may be configured to emit electromagnetic radiation at multiple different levels of intensity. Correspondingly, electromagnetic radiation may impinge on one or more eyes and/or eyelids of subject 106 at multiple different levels of intensity. The level of exposure for subject 106, for some types and/or stages of light therapy, in particular with regard to impingement as described herein, may be determinative for stimulation of the S-cone receptors and/or modifications of one or more characteristics of the circadian rhythm of subject 106. Light source 30 may be configured to emit electromagnetic radiation having multiple different wavelengths and/or other characteristic parameters for different types and/or stages of light therapy.

For example, for some types and/or stages of light therapy, the levels of intensity may include, at least and without limitation, a first level and a second level. The multiple levels of intensity may alternate and/or be used in sequence. The second level of intensity may be lower than the first level of intensity. Light source 30 may be configured to alternate between multiple levels of intensity to pulse electromagnetic radiation, for example pulsing electromagnetic radiation between a first level and a second level of intensity. One of the levels of intensity, *e.g.* the second level, may be zero or near-zero emission of electromagnetic radiation. One of the levels of intensity, for some types and/or stages of light therapy, may be sufficient to stimulate the S-cone receptors of the eye of the subject upon impingement. Light source 30 may be configured, for some types and/or stages of light therapy, to pulse electromagnetic radiation between an intensity that is sufficient to stimulate the S-cone receptors of the eye of the subject and a different, lower intensity.

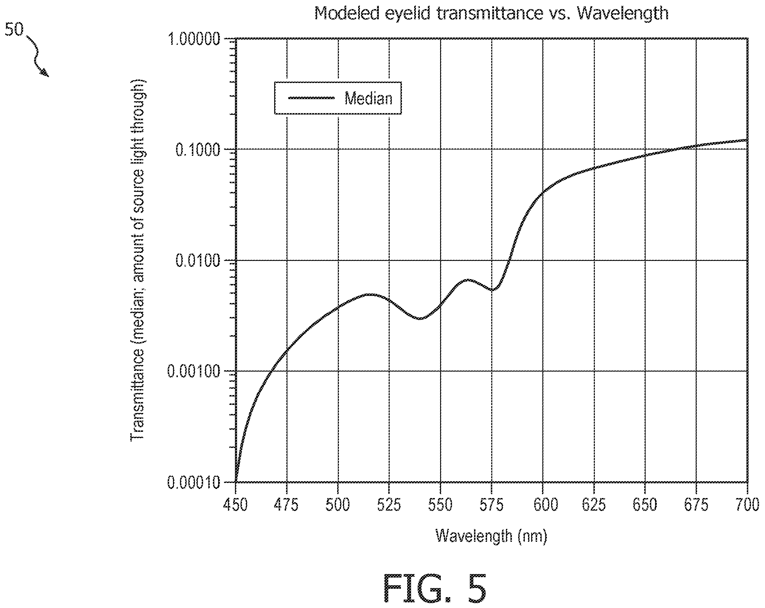

By way of non-limiting example, exposure for one hour, through closed eyelids of a subject, to pulses of two seconds of electromagnetic radiation per minute, delivered through a light mask, the electromagnetic radiation having a wavelength of peak intensity about 480 nm, a full-width-half-maximum of about 24 nm, and a level of intensity of about 111 W/m², have been shown to delay circadian phase and suppress nocturnal melatonin. This electromagnetic radiation has a mean corneal irradiance level of about 0.31 W/m² for an eyelid transmittance of about 0.0028 at 480 nm. Corneal irradiance levels may be based on a calculated circadian stimulus (CS) value. Circadian stimulus may be equivalent to the estimated percentage of light-induced nocturnal melatonin suppression following one hour exposure to the retina (open eyes) from a non-flashing light source through a fixed pupil of 2.3 mm; in this case a CS value of 0.34. Melatonin suppression as a function of circadian stimulus corresponds to a known function. The spectral sensitivity of the human circadian system to narrow-band spectra based upon nocturnal melatonin suppression corresponds to a known function. Sufficient levels of exposure, at a closed eyelid, the cornea, or the retina of a subject, for electromagnetic radiation having a peak intensity at a different wavelength than described in this paragraph, and/or having a different full-width-half-maximum than described in this paragraph, may be determined by scaling according to a spectral sensitivity function (see, by way of non-limiting example, FIG. 5). Note that measurements of the level of intensity in this example were taken at about 39 mm in front of the light mask, corresponding to an estimate of the location of a subject's eyelid, which may vary due to anatomical differences. Retinal mechanisms responsible for photic stimulation of the circadian system may be approximated through a model of human circadian phototransduction, which may be subject to refinement responsive to future research.

Individual light sources 30 may include one or more of a light-emitting diode (LED), organic LED (OLED), and/or other source of electromagnetic radiation, in particular non-parallel or non-focused electromagnetic radiation. In some embodiments, the level of exposure to a closed eyelid and/or a retina of a subject may correspond to about 1 W/m², about 10 W/m², about 100 W/m², about 1000 W/m², and/or another level of intensity for electromagnetic radiation that is sufficient to stimulate, *e.g.*, the S-cone receptors of the eye of the subject upon impingement. For some types and/or stages of light therapy, the electromagnetic radiation may be within a range of wavelengths ranging between about 410 nm and about 510 nm.

The emitted electromagnetic radiation may not be narrow or focused in the way, *e.g.*, a laser would be. Rather, the emitted electromagnetic radiation may have a wide angle (*e.g.* more than about 10 degrees, about 15 degrees, about 20 degrees, and/or another number of degrees). Alternatively, and/or simultaneously, the emitted electromagnetic radiation 21 may be diverging to a predetermined degree (*e.g.* such that the diameter of the cross-section of a cone of electromagnetic radiation emitted from an individual light source 30 disposed near the eye of subject 106 is more than about 0.5 cm, about 1 cm, about 2 cm, about 3 cm, about 5 cm, and/or other diameters at a distance less than 2 cm from an individual eye or eyelid of subject 106, and/or other combinations of a diameter of a cross-section of a cone and distance that correspond to a similar degree of diverging electromagnetic radiation). Alternatively, and/or simultaneously, the emitted electromagnetic radiation may be diffuse, and/or otherwise not narrow. Light sources 30 may be arranged in a regular pattern, irregular pattern, or combination of both. For example, light sources 30 may be arranged in a regular grid as depicted in FIG. 4.

Returning to FIGs. 1-3, light sources 30 of system 10 may be configured to have a controllable level of intensity (*e.g.* denoted by a percentage of the maximum available level of intensity for an individual light source), a controllable direction and/or angle of illumination, a controllable selection of illumination spectra, a controllable selection of wavelength bands, and/or other controllable illumination characteristics and/or illumination parameters. For example, illumination parameters of a light source 30 may be controlled by adjusting optical components within the light source, including, but not limited to, one or more of refractive components, reflective components, lenses, mirrors, filters, polarizers, diffraction gradients, optical fibers, and/or other optical components. Individual light sources 30 may be controlled such that only part of subject 106 is illuminated. As used herein, "illumination" of subject 106 may be interchangeably referred to as impingement of electromagnetic radiation on subject 106.

Note that electromagnetic radiation emitted by real-world light sources, as opposed to theoretical models of light sources, may have a non-deterministic distribution of its intensity and/or (beam) direction, at least for practical applications of phototherapy and/or digital image processing. Nonetheless, electromagnetic radiation may be considered to "substantially" directly impinge on or near a particular surface and/or location if at least about 90%, at least about 95%, about 99%, and/or another percentage of the emitted radiation directly so impinges. As used herein, electromagnetic radiation may be considered to be "substantially" in a particular band of wavelengths if at least about 50%, about 75%, about 90%, about 95%, about 99%, and/or another percentage of the emitted electromagnetic radiation has a wavelength in the particular band of wavelengths. It may be preferred that more than about 90% of the spectral power is in the 450 - 510 nm band, and less than 10% power is above 510 nm. Spectral power above a certain wavelength may act to reduce the Retinal Ganglion Cell (RGC) stimulation function of the S-cone (or blue cone). Retinal Ganglion Cell (RGC) stimulation may be affected through color opponency within the human visual system. Broad-band and multi-band lights can be as effective as selected narrow-band sources, but with lower efficacy.

In some embodiments, for some types and/or stages of light therapy, the emitted electromagnetic radiation consists substantially of electromagnetic radiation in a narrow wavelength band. As used herein, a narrow wavelength band may be defined as spanning (between the highest and lowest wavelengths in a particular narrow band) about 70 nm, about 60 nm, about 50 nm, about 40 nm, about 30 nm, about 20 nm, about 10 nm, about 5 nm, and/or other wavelength bands. In some embodiments, the emitted electromagnetic radiation consists substantially of blue light. In some embodiments, the emitted electromagnetic radiation consists substantially of electromagnetic radiation in a particular wavelength band. The particular wavelength band may be from about 410 nm to about 510 nm, about 400 nm to about 500 nm, about 430 nm to about 490 nm, from about 450 nm to about 500 nm, from about 460 nm to about 490 nm, from about 430 nm to about 510 nm, from about 460 nm to about 510 nm, and/or other particular wavelength bands.

System 10 may include an eyelid detector (see FIG. 4, item 41) configured to determine whether an eye of the subject is closed, whether sleep mask 12 is taken off, whether subject 106 is awake, and/or other determinations. Either circumstance (or any combination thereof) may be a justification to turn off light source(s) 30 for any lighting modules in use, and/or change any other operating parameter of system 10. By way of non-limiting example, both lighting modules in FIG. 4 each comprise four light sources 30.

System 10 may operate with increased efficiency if light source 30 has less spectral power in bands outside of the response curve of the S-cone. The S-cone has maximum responsiveness in a particular spectral band. In some embodiments, a majority, if not all, spectral power may be in the S-cone response band. For example, in some embodiments, light source 30 may use narrow-band light which has dominant wavelengths in the S-cone response band. In some embodiments, for subjects having closed eyelids, the delivery of light having longer wavelengths in the S-cone response band may be preferred, *e.g.* in the wavelength range between about 450 nm and about 490 nm.

As can be seen in FIG. 1, shield 13 may include first shield portion 20 and second shield portion 22. First shield portion 20 is configured to cover a first eye of the subject. Second shield portion 22 is configured to cover a second eye of the subject. In order to comfortably cover the first eye and the second eye of the subject, first shield portion 20 and second shield portion 22 are substantially larger than the ocular openings of the eyes of subject 106.

In certain embodiments, first shield portion 20 and second shield portion 22 may be joined by connecting shield portion 24. Connecting shield portion 24 may be configured to rest on at least a portion of the nose of the subject (*e.g.*, across the bridge of the nose) when the subject is wearing sleep mask 12. In some instances (not shown), connecting shield portion 24 may be narrower or thicker than the embodiment depicted in FIGs. 1-3.

In certain embodiments, shield 13 is formed from flexible materials. The flexibility of shield 13 may enhance the comfort of shield 13 to the subject. The side of shield 13 visible in FIG. 3 faces toward the subject during use. On this side, a base surface 26 substantially impermeable to liquids may be formed. For example, the impermeable base surface 26 may be formed by a flexible plastic material such as polycarbonate, polyester, and/or other materials. The impermeability of base surface 26 may protect electronic components of sleep mask 12 carried within shield 13 from moisture.

In certain embodiments, shield 13 may include cushioning layer 28 disposed on base surface 26. Cushioning layer 28 may be formed from a soft, resilient material. For example, cushioning layer 28 may be formed from foam, fabric, fabric/foam laminate, and/or other materials. During use, cushioning layer 28 provides the innermost surface to the subject, and engages the face of the subject. As such, the softness of cushioning layer 28 provides a cushion for the face of the subject, and enhances the comfort of sleep mask 12 to the subject.

As will be appreciated from the foregoing and FIGs. 1-3, during use shield 13 may provide a barrier between ambient radiation and one or more eyes of subject 106. In certain embodiments, shield 13 is opaque, and blocks ambient radiation (at least within the visible spectrum), thereby shielding the eyes of subject 106 from ambient radiation.

Strap 14 is configured to hold shield 13 in place on the subject. In the embodiments shown in FIGs. 1-3, strap 14 is attached to each of first shield portion 20 and second shield portion 22, and wraps around the head of the subject to hold sleep mask 12 in place on the head of the subject. Strap 14 may be adjustable in length (*e.g.*, to accommodate different sized heads). Strap 14 may be formed from a resilient material (*e.g.*, elastic) that stretches to accommodate the head of the user and holds shield 13 in place. It should be appreciated that the inclusion of strap 14 in the embodiments of sleep mask 12 illustrated in FIGs. 1-3 is not intended to be limiting. Other mechanisms for holding appliance 11, sleep mask 12, and/or shield 13 in place on the subject (on, near, around, and/or in one *or* both eyes) are contemplated. For example, a more elaborate headgear may be implemented (such as a full face-mask or an ear-mounted structure), an adhesive surface may be applied to shield 13 that removably adheres to the skin of the subject to hold or mount shield 13 in place (see *e.g.* FIG 6B, in which an adhesive surface of mounting feature 62 removably adheres to the skin of subject 60), a rigid or flexible frame (such as eyeglasses or frames that similarly rest on the side of the face and/or the ears, see *e.g.* FIG. 6C, in which rigid frame 61 is worn by subject 60 in a manner that allows emission of radiation near the eye of subject 60), and/or other mechanisms for holding shield 13, lighting module 16, lighting module 18, light source(s) 30, and/or other components of appliance 11 in place may be implemented. See *e.g.* FIG. 6A, in which appliance 63 adheres to or is held onto the head of subject 60 around the eyes. In certain embodiments, such as illustrated in FIG. 6C, rigid frame 61 configured to carry a light source may not completely obscure the subject's vision.

As depicted in FIG. 3, first lighting module 16 and second lighting module 18 may be mounted to first shield portion 20 and second shield portion 22, respectively, on the side of shield 13 that faces toward the face of the subject during use. First lighting module 16 and second lighting module 18 may be backlit, and are configured to emit, guide, deflect, filter, and/or diffuse electromagnetic radiation such that the electromagnetic radiation impinges on the face of subject 106 and/or about the eyes of subject 106. The electromagnetic radiation emitted by first lighting module 16 and second lighting module 18 has a wavelength (and/or wavelengths, or wavelength band, or range of wavelengths) that have an impact on the (sleeping) subject, when they are delivered in accordance with the intended operation described herein. In some instances, the electromagnetic radiation emitted by first lighting module 16 and second lighting module 18 is directed towards the eyes or eyelids of the subject in radiation fields having relatively uniform luminance as perceived by the subject. For example, in one embodiment, the luminance of the electromagnetic radiation emitted by first lighting module 16 and second lighting module 18 varies across the respective emitted fields by an amount that is less than or equal to about 100:1 for use with eyes open, and less than 10,000:1 for eyes-closed applications. The size of the uniform field of radiation formed by either first lighting module 16 or second lighting module 18 may correspond to the size of the eye of subject 106. Electromagnetic radiation from light source(s) 30 may be guided through a (waveguide) layer, component, and/or module that diffuses, (re)directs, (optionally/temporarily) blocks, and/or filters the electromagnetic radiation before it reaches subject 106.

An example of a sleep mask implementing one or more stated functions of sleep mask 12 is disclosed in United State Patent Application 61/141,289, titled "System and Method for Administering Light Therapy", filed December 30, 2008, which is hereby incorporated by reference herein in its entirety.

By way of illustration, FIG. 5 provides a plot 50 of transmittance of the human eye lid (on a logarithmic scale) versus wavelength. As can be seen in FIG. 5, transmittance through the eyelid varies based on wavelength. As such, although electromagnetic radiation at 460 nm may use peak sensitivity of an open eye to shift the sleep cycle when directing electromagnetic radiation to an open eye, attenuation of electromagnetic radiation at 460 nm may reduce the efficiency of this wavelength when electromagnetic radiation is being provided to the eye of the subject through the eyelid (*e.g.*, as is done by the sleep mask 12 shown in FIGs. 1-3). As is shown in FIG. 5, light in the range of about 450 nm to 550 nm may be attenuated by a factor of ten (10) or greater than longer wavelength light (*e.g.* wavelengths greater than 575 nm) as light passes through the eyelids.

FIG. 5 illustrates through curve 50 that radiation below a wavelength of approximately 590 nm exhibit a markedly lower transmittance through a closed eyelid than a wavelength of over 600 nm. To compensate accordingly for this varying transmittance, electromagnetic radiation may be emitted using or having more power as needed. Certain embodiments may use a wavelength in the range of visible light, in the range of invisible light which is converted to a visible light (such as by phosphorescence), a wavelength having substantial power in visible wavelengths greater than 430 nm, a wavelength having substantial power in visible wavelengths greater than 460 nm, a wavelength having substantial power in visible wavelengths greater than 490 nm, a wavelength having substantial power in visible wavelengths between 460 nm and 490 nm, a wavelength having substantial power in visible wavelengths between 450 nm and 500 nm, wavelengths used by a plurality of relatively monochromatic light sources inside any of the stated wavelengths and wavelength bands in the present specification, time-varying patterns of radiation used to wake the subject, alter a sleep stage, and/or cause relaxation, and/or other wavelengths. Common approaches to modification of the circadian rhythm through the provision of light therapy to closed eyes have suffered from inaccurate data about eyelid attenuation and/or partial understanding of the human non-visual light-response-related systems, in addition to the practical problems of power requirement and power dissipation as described elsewhere herein.

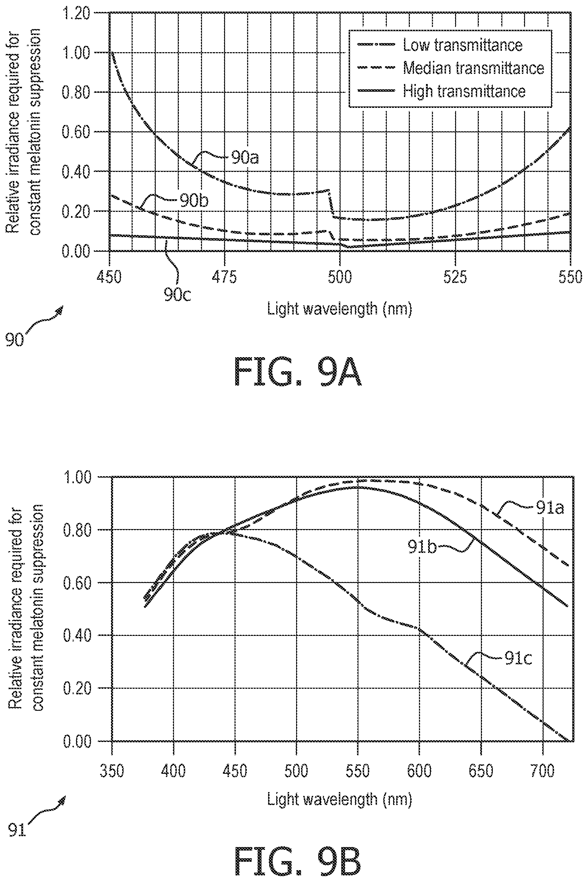

By way of illustration, FIG. 9A illustrates graph 90, depicting the relative irradiance (of monochromatic light) required for constant melatonin suppression versus wavelength for three categories of subjects: low (90a), medium (90b), and high (90c) transmittance. Note that the range among categories of subjects, approximately 10:1, indicates that light therapy may be patient-specific, and may need to be individually tailored for effectiveness. The preferred embodiment as described elsewhere may not be considered efficient in light of FIG. 9A and may, as a result, not be used in common approaches to modification of the circadian rhythm.

By way of illustration, FIG. 9B illustrates graph 91, depicting relative physiological response (*e.g.* of retinal cone receptors), normalized to the L-cone maximum, versus wavelength for red, green, and blue cone receptors (graphs 91a, 91b, and 91c, respectively). The light-stimulation response of retinal cone receptors (*e.g.* about 10 ms) is faster than the response for direct stimulation of the intrinsically photosensitive Retinal Ganglion Cells (*e.g.* about 15 minutes). The time for the respective RGC activity to substantially decay corresponds accordingly from about 30 to 120 minutes. While some approaches may use or prefer a wavelength band of about 430 nm to about 490 nm for opened eyes, the preferred blue cone receptor response for closed eyes may include a wavelength band of about 460 nm to about 490 nm.

Once emission of radiation is initiated, the radiation may follow a combination, pattern, and/or sequence whereby different light sources within a lighting module use different wavelengths, different wavelength bands, different levels of illuminance, or any (sequential) combination thereof. For example, the level of illuminance may be gradually increased to gently wake up subject 106 at an appropriate moment.

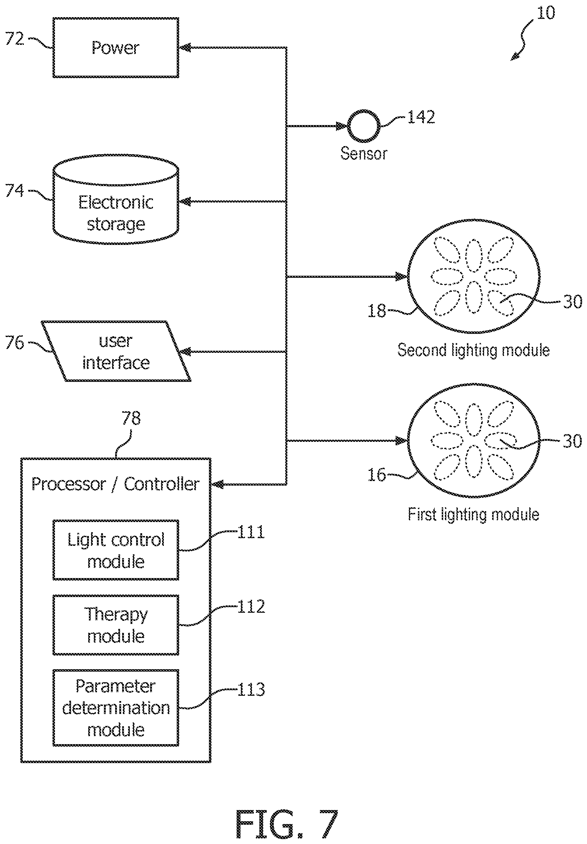

FIG. 4 illustrates system 10 and, in particular, first lighting module 16 in additional detail. First lighting module 16 may include one or more light sources 30. The embodiment depicted on the right-hand side of FIG. 4 includes four light sources 30 per lighting module, whereas the more detailed depiction of first lighting module 16, on the light-hand side of FIG. 4 includes a set, array, and/or grid of approximately six by four light sources 30 and a communication connector 42 configured such that other components of system 10 can communicate with and/or control first lighting module 16. Note that any depictions, including FIG. 7 are merely exemplary and not intended to be limiting in any way. Sleep mask 12 may be controlled and/or powered wirelessly, thus obviating the need for wires that connect sleep mask 12 to, *e.g.*, a processor or a power source.

FIG. 7 schematically illustrates various components of system 10 according to one or more embodiments. As can be seen in FIG. 7, in addition to one or more of the components shown in FIGs. 1-3 and described above, system 10 may include one or more of a power source 72, one or more sensors 142, electronic storage 74, a user interface 76, a processor/controller 78 (with regard to FIG. 7 and elsewhere herein referred to as processor 78), various computer program modules, and/or other components. The computer program modules may include a light control module 111, a therapy module 112, a parameter determination module 113, and/or other modules. In some embodiments, one or more of the depicted components in FIG. 7 may be carried on shield 13 and/or strap 14 of sleep mask 12 (illustrated in FIGs. 1-3), for example through being removably attached and/or disconnectable from the rest of sleep mask 12. This will enable those components to be removed from a given shield 13 and/or strap 14, and attached to another shield 13 and/or strap 14, which may be beneficial if shield 13 and/or strap 14 degrade over time and/or with usage and must be replaced. Power source 72, electronic storage 74, user interface 76, and/or processor 78 may control operation of one or more light sources 30 associated with first lighting module 16 and/or second lighting module 18, as is discussed below. Note that the depiction of eight light sources 30 per light module in FIG. 7 is exemplary and not intended to be limiting in any way.

Power source 72 provides the power to operate one or more components of system 10, including but not limited to light sources 30 associated with first lighting module 16 and second lighting module 18, electronic storage 74, user interface 76, and/or processor 78. Power source 72 may include a portable source of power (*e.g.*, a battery, a fuel cell, *etc.*), and/or a non-portable source of power (*e.g.,* a wall socket, a large generator, *etc.*). In one embodiment, power source 72 includes a portable power source that is rechargeable. In one embodiment, power source 72 includes both a portable and non-portable source of power, and the subject is able to select which source of power should be used to provide power to system 10. The level of power required to operate system 10 depends in part of the level of illuminance used for light source(s) 30. Lower required power levels may correspond to smaller and/or cheaper batteries. By carefully selecting the wavelength or band of wavelengths used in lighting modules by light source(s) 30, a low level of illuminance and thus power, *e.g.* 20 to 30 lux for open eyes (and/or a corresponding amount of irradiance) which may correspond to approximately 50 mW using certain types of LEDs, may be adequate for the purposes described herein.

In one embodiment, electronic storage 74 comprises electronic storage media that electronically stores information. The electronic storage media of electronic storage 74 may include one or both of system storage that is provided integrally (*i.e.*, substantially non-removable) with system 10 and/or removable storage that is removably connectable to system 10 via, for example, a port (*e.g.,* a USB port, a FireWire port, *etc.*) or a drive (*e.g.*, a disk drive, *etc.*). Electronic storage 74 may include one or more of optically readable storage media (*e.g.*, optical disks, *etc.*), magnetically readable storage media (*e.g.*, magnetic tape, magnetic hard drive, floppy drive, *etc.*), electrical charge-based storage media (*e.g.*, EPROM, EEPROM, RAM, *etc.*), solid-state storage media (*e.g.*, flash drive, *etc.*), and/or other electronically readable storage media. Electronic storage 74 may store software algorithms, information determined by processor 78, information received via user interface 76, and/or other information that enables system 10 to function properly. For example, electronic storage 74 may record or store one or more illumination parameters (as discussed elsewhere herein), and/or other information. Electronic storage 74 may be a separate component within system 10, or electronic storage 74 may be provided integrally with one or more other components of system 10 (*e.g.*, processor 78).

User interface 76 is configured to provide an interface between system 10 and a user (or medical professional, or other device, or other system) through which the user can provide and/or receive information. This enables data, results, and/or instructions and any other communicable items, collectively referred to as "information," to be communicated between the user and system 10. An example of information that may be conveyed to a subject is the current time, or a scheduled wake-up time. Other examples of information that may be conveyed are: circadian rhythm related information like phase and/or intensity, or user performance related information like scheduled physical or mental performance events. Examples of interface devices suitable for inclusion in user interface 76 include a keypad, buttons, switches, a keyboard, knobs, levers, a display screen, a touch screen, speakers, a microphone, an indicator light, an audible alarm, and a printer. Information may be provided to the subject by user interface 76 in the form of auditory signals, visual signals, tactile signals, and/or other sensory signals.

By way of non-limiting example, user interface 76 may include a light source capable of emitting light. The light source may include, for example, one or more of at least one LED, at least one light bulb, a display screen, and/or other sources. User interface 76 may control the light source to emit light in a manner that conveys to the subject information related to operation of system 10. Note that subject 106 and the user of system 10 may be one and the same person.

It is to be understood that other communication techniques, either hardwired or wireless, are also contemplated herein as user interface 76. For example, in one embodiment, user interface 76 may be integrated with a removable storage interface provided by electronic storage 74. In this example, information is loaded into system 10 from removable storage (*e.g.*, a smart card, a flash drive, a removable disk, *etc.*) that enables the user(s) to customize the implementation of system 10. Other exemplary input devices and techniques adapted for use with system 10 as user interface 76 include, but are not limited to, an RS-232 port, RF link, an IR link, modem (telephone, cable, Ethernet, internet or other). In short, any technique for communicating information with system 10 is contemplated as user interface 76

One or more sensors 142 of system 10 in FIG. 7 may be configured to generate output signals conveying information related to physiological, environmental, and/or patient-specific (medical) parameters related to subject 106, and/or other information. System 10 may use any of the generated output signals to monitor subject 106. In some embodiments, the conveyed information may be related to parameters associated with the state and/or condition of subject 106, the breathing of subject 106, the gas breathed by subject 106, the heart rate of subject 106, the respiratory rate of subject 106, vital signs of subject 106, including one or more temperatures, oxygen saturation of arterial blood (SpO₂), whether peripheral or central, and/or other parameters.

As a non-limiting example, one or more sensors 142 may generate one or more output signals conveying information related to a location of subject 106, *e.g.* through stereoscopy. The location may be a three-dimensional location of subject 106, a two-dimensional location of subject 106, a location of a specific body part of subject 106 (*e.g.,* eyes, arms, legs, a face, a head, a forehead, and/or other anatomical parts of subject 106), the posture of subject 106, the orientation of subject 106 or one or more anatomical parts of subject 106, and/or other locations. In some embodiments, one or more sensors 142 may be configured to generate output signals conveying information related to whether the eyes of subject 106 are opened or closed, and/or which direction the eyes of subject 106 are facing. During light therapy, it may be preferred that emitted electromagnetic radiation from light sources 30 substantially does not directly impinge on open eyes of subject 106. Sensors 142 may include one or more of a temperature sensor, one or more pressure/weight sensors, one or more light sensors, one or more electromagnetic (EM) sensors, one or more infra-red (IR) sensors, one or more still-image cameras, one or more video cameras, and/or other sensors and combinations thereof.

The illustration of sensor 142 including one member in FIG. 7 is not intended to be limiting. System 10 may include one or more sensors. The illustration of a particular symbol or icon for sensor 142 in FIG. 1 is exemplary and not intended to be limiting ion any way. Resulting signals or information from one or more sensors 142 may be transmitted to processor 78, user interface 76, electronic storage 74, and/or other components of system 10. This transmission can be wired and/or wireless.

Processor 78 is configured to provide information processing and/or system control capabilities in system 10. As such, processor 78 may include one or more of a digital processor, an analog processor, a digital circuit designed to process information, an analog circuit designed to process information, and/or other mechanisms for electronically processing information. In order to provide the functionality attributed to processor 78 herein, processor 78 may execute one or more modules. The one or more modules may be implemented in software; hardware; firmware; some combination of software, hardware, and/or firmware; and/or otherwise implemented. Although processor 78 is shown in FIG. 7 as a single entity, this is for illustrative purposes only. In some implementations, processor 78 may include a plurality of processing units. These processing units may be physically located within the same device (*e.g.*, a sleep mask), or processor 78 may represent processing functionality of a plurality of devices operating in coordination.

As is shown in FIG. 7, processor 78 is configured to execute one or more computer program modules. The one or more computer program modules include one or more of light control module 111, therapy module 112, parameter determination module 113, and/or other modules. Processor 78 may be configured to execute modules 111-113 by software; hardware; firmware; some combination of software, hardware, and/or firmware; and/or other mechanisms for configuring processing capabilities on processor 78.

It should be appreciated that although modules 111-113 are illustrated in FIG. 7 as being co-located within a single processing unit, in implementations in which processor 78 includes multiple processing units, one or more of modules 111-113 may be located remotely from the other modules. The description of the functionality provided by the different modules 111-113 described below is for illustrative purposes, and is not intended to be limiting, as any of modules 111-113 may provide more or less functionality than is described. For example, one or more of modules 111-113 may be eliminated, and some or all of its functionality may be provided by other ones of modules 111-113. Note that processor 78 may be configured to execute one or more additional modules that may perform some or all of the functionality attributed below to one of modules 111-113.

Parameter determination module 113 of system 10, depicted in FIG. 7, may be configured to determine one or more status parameters, medical parameters, and/or other parameters from output signals generated by one or more sensors 142. Parameters may be related to a subject's physiological, environmental, and/or patient-specific parameters. One or more status parameters may be related to whether one or more eyes of subject 106 are open or closed. For example, such a status parameter may be determined based on one or more output signals of eyelid detector 41 (shown in FIG. 4). One or more medical parameters may be related to monitored vital signs of subject 106, and/or other medical parameters of subject 106. For example, one or more medical parameters may be related to whether subject 106 is awake or asleep, or, in particular, what the current sleep stage of subject 106 is. Other parameters may be related to the environment near system 10, such as, *e.g.*, air temperature, ambient noise level, ambient light level, and/or other environmental parameters. One or more physiological parameters may be related to and/or derived from EEG measurements, EMG measurements, respiration measurements, cardiovascular measurements, HRV measurements, ANS measurements, and/or other measurements. Some or all of this functionality may be incorporated or integrated into other computer program modules of processor 78.

Light control module 111 of system 10 in FIG. 7 is configured to control emission of one or more light sources 30. By controlling impingement of electromagnetic radiation on subject 106, system 10 provides light therapy to subject 106. Controlling impingement may include controlling one or more light sources 30 based on one or more determined (operating) parameters. Control by light control module 111 may be based on individual light sources 30, one or more subsets of light sources 30, one or more groups of light sources 30, one or more rows and/or columns of light sources 30, and/or any combination thereof. Control by light control module 111 may include control of the controllable level of intensity, the controllable direction and/or angle of illumination, the controllable selection of illumination spectra, and/or other controllable illumination characteristics and/or illumination parameters of one or more light sources 30. Control by light control module 111 may be based on information from parameter determination module 113.

Substantially continuous emission of electromagnetic radiation at a level of intensity that is sufficient to modify one or more characteristics, in particular the phase, of the circadian rhythm of subject 106 commonly suffers from the practical problems of power requirement and power dissipation (despite being otherwise effective in stimulating the ipRGCs at appropriate wavelengths, including, but not limited to, about 530 nm). The power requirements may be above and beyond the limitations of portable power sources. The related power dissipation may cause a heat transfer to subject 106, and may in turn be uncomfortable, even prohibitively so. As used herein, "substantially continuous emission of electromagnetic radiation" includes emission that pulses so rapidly that the effects on a subject (including the eyes and the visual systems of the subject, in particular the response time of various visual and non-visual receptors) are the same or similar as continuous emission, because of the integration property of these receptors; afforded by rapid response and slower decay.

Some approaches to light therapy using substantially continuous electromagnetic radiation in a narrow wavelength band, with direct unfiltered access to the cornea, have commonly focused on the wavelength band between about 450 nm and about 475 due to the sensitivity profile in that wavelength band. Even if such an approach were able, somehow, to overcome the practical problems described above (and that appears to not be the case using current and common technology), such an emission would cause a substantial suppression of the level of melatonin production, thus at a potentially inopportune time, making it difficult for the subject to fall asleep and/or remain asleep. Some such approaches have focused on the wavelength band between about 500 nm and about 530 nm as the band for continuous light activation of the intrinsically photosensitive Retinal Ganglion Cells. Note that substantial suppression of the level of melatonin production may be undesirable for various reasons.

Some approaches to light therapy using pulses of electromagnetic radiation have commonly focused on using white light. Commonly, such approaches use pulse periods as brief as 2 ms. Even if such an approach were able, somehow, to overcome the practical problems described above (and that appears to not be the case using current and common technology), and simultaneously shift the circadian rhythm of a subject, such light therapy causes a substantial suppression of the level of melatonin production, thus awakening the subject at a potentially inopportune time, and/or making it difficult for the subject to fall asleep and/or remain asleep. Some approaches to light therapy use exposure to red light, which may cause an alertness response. It is hypothesized such results may be caused by the release of Cortisol in response to the impingement of red light. It is noted that red light (wavelengths longer than approximately 600 nm) causes affectively no suppression of the level of melatonin production, nor a shift of the circadian rhythm of a subject.

In some embodiments, light control module 111 may be configured to control emission of one or more light sources 30 such that, for some types and/or stages of light therapy, substantially blue light is emitted in particular pulses to provide light therapy and/or modify one or more characteristics of the circadian rhythm of subject 106. The particular wavelength band of blue light may be from about 430 nm to about 490 nm, from about 450 nm to about 500 nm, from about 460 nm to about 490 nm, from about 430 nm to about 510 nm, from about 460 nm to about 510 nm, and/or other particular wavelength bands. The pulses of blue light may have particular characteristics, including a particular pulse duration, a particular inter-pulse duration, a particular wavelength (band), a particular level of intensity or power level, and/or other characteristics. The particular pulse duration may be controlled to be about 1 ms, about 10 ms, be about 100 ms, be between about 0.5 seconds and about 1 minute, between about 30 seconds and about 2 minutes, about 1 minute, about 10 minutes, and/or other pulse durations. The particular inter-pulse duration may be controlled to be about 1 ms, about 10 ms, be about 100 ms, be between about 0.5 seconds and about 1 minute, between about 30 seconds and about 2 minutes, about 1 minute, about 10 minutes, and/or other inter-pulse durations. In some embodiments, the one or more light sources 30 emit blue light at an intensity sufficient to stimulate the S-cone receptors for about 2 seconds out of every minute during the provision of light therapy. The usage of pulsed electromagnetic radiation as described herein, in particular in a predetermined narrow wavelength band as described elsewhere herein, may shift the phase of the circadian rhythm to a similar degree as substantially continuous electromagnetic radiation does, but in a manner that avoids, reduced, and/or minimizes the practical problems described above.

The waveform (or shape) of the pulses may be a sinusoid, square, triangular, saw-tooth, and/or other waveform, and/or any combination thereof. In some embodiments, square and/or imperfectly square waveforms may be preferred. Imperfectly square waveforms may be referred to as pseudo-square waveforms. Practical limitations may prevent an ideal square waveform. Rise time and fall time for a waveform may be defined as the time for a signal to transition between 10 % and 90 % of a target level, and *vice versa*, respectively. Usually, the target level is the highest level a signal will reach. In some cases, the target level may be the average and/or aggregate level during a particular phase of the period. As used herein, for some types and/or stages of light therapy, the target level may be the intensity (or magnitude) that is sufficient to stimulate the S-cone receptors. In some embodiments, the waveform of the pulses may be a square or pseudo-square waveform having rise and/or fall times of about 0.01 ms, about 0.1 ms, about 1 ms, about 10 ms, about 100 ms, about 1 second, and/or other rise and fall times. In some embodiments, the waveform of the pulses may be a square or pseudo-square waveform having rise and/or fall times of about 0.01% of the pulse duration, about 0.1% of the pulse duration, about 1% of the pulse duration, about 10% of the pulse duration, and/or another percentage of the pulse duration. In some embodiments, the rise time and the fall time may be different. For example, the rise time may be shorter than the fall time.

In some embodiments, light control module 111 is configured such that the emitted pulses of electromagnetic radiation provide light therapy by modifying and/or shifting one or more characteristics of the circadian rhythm of subject 106. By way of non-limiting example, the provided light therapy may shift the phase of the circadian rhythm of subject 106. In particular, the phase of the circadian rhythm may be shifted in such a way that a level of melatonin production by subject 106 is not substantially suppressed. As used herein, "not substantially suppressing" a level of melatonin production means only suppressing melatonin production by 1/5 to 1/3 as much as substantially continuous blue light would, and/or only suppressing melatonin production by an amount that is unlikely and/or insufficient to wake up subject 106 in a manner that the suppression of melatonin production commonly acts to wake up subject 106 at or near the end of the sleep cycle. A modification or shifting of the phase of the circadian rhythm may correspond to a modification of melatonin suppression. For example, shifting the phase of the circadian rhythm by 30 minutes may shift the onset of melatonin suppression (and thus the related awakening time) by about 30 minutes as well.

In some embodiments, controlling impingement by light control module 111 may include adjusting modifiable physical/mechanical structures, *e.g.* apertures, to block or partially block electromagnetic radiation. Alternatively, and/or simultaneously, a structure or object placed between one or more light sources 30 and one or more eyes of subject 106 may accomplished the same functionality. For example, an array of liquid crystals, such as may be included in a liquid crystal display (LCD), may be selectively controlled to adjust how much electromagnetic radiation passes through (*e.g.* in the direction from one or more light source 30 to subject 106).

In some embodiments, light control module 111 may be configured to control and/or dictate timing parameters of the electromagnetic radiation emitted by first lighting module 16 and second lighting module 18 toward the face of the subject on or about the eyes of the subject. The timing parameters may be based on the particular pulse duration, inter-pulse duration, and/or other timing-related characteristics of the emitted electromagnetic radiation. For example, using a pulse duration of 3 seconds and an inter-pulse duration of 97 seconds, light control module 111 may be configured to turn on one or more light sources 30 at a start time, turn off the one or more light sources 30 responsive to 3 seconds having elapsed, waiting 97 seconds while the one or more light sources are turned off, and repeating this cycle for the predetermined period of recommended light therapy. In some embodiments, one or more aspects of the operation of system 10 may be adjusted or customized per individual subject. Adjustments and/or customizations may be input to system 10 via user interface 76. In one embodiment, electronic storage 74 stores a plurality of different patterns of emission of electromagnetic radiation, and subject 106 (and/or a caregiver) select the appropriate pattern for subject 106 via user interface 76. In some implementations, a pattern including one or more particular emissions of electromagnetic radiation may cover a 24-hour period, and/or be repeated daily. In some implementations, a pattern may span one or more days. For example, a shift worker may have a first type of electromagnetic radiation and/or treatment during the work week, and a second type of treatment during the weekend. Alternatively, and/or simultaneously, a shift worker may use transitional types of treatments to aid transition between the first type and the second type.

In certain embodiments, a light therapy regimen may dictate the timing of the administration of electromagnetic radiation to subject 106 by system 10. In such embodiments system 10 may include a clock and/or timer (referred to herein as "clock"). Light control module 111 may operate in conjunction and/or based on the clock. The clock may be capable of monitoring elapsed time from a given event and/or of monitoring the time of day. Subject 106 (and/or a caregiver) may be enabled to correct the time of day generated by the clock via, for example, user interface 76. The emitted electromagnetic radiation may include gradually incrementing the level of illuminance (or radiation intensity) over time, *e.g.* based on the clock. For example, the level of illuminance could be increased every minute (capped by a maximum level of illuminance) until, *e.g.*, the subject wakes up. The emitted electromagnetic radiation may similarly change the wavelength of the radiation over time, *e.g.* based on the clock. Furthermore, multiple types or varieties of emitted electromagnetic radiation may be combined in some sequence.

Therapy module 112 is configured to determine and/or obtain a light therapy regimen for subject 106. For example, a medical professional, such as a doctor, may provide a light therapy regimen. Therapy module 112 may be configured to determine a (recommended) phototherapy regimen for subject 106, for example based on one or more parameters such as parameters determined by parameter determination module 113. A light therapy regimen may define one or more stages of light therapy. Individual stages may be associated with an individual period during one or more days. For example, a particular period for a particular light therapy may be an hour before the subject needs to wake up. In some implementations, multiple stages of light therapy for a particular light therapy regimen may be associated with mutually exclusive periods. In some implementations, multiple stages may (*e.g.* partially) overlap.

In some implementations, multiple stages of light therapy for a particular light therapy regimen may use different wavelengths, wavelength ranges, pulses or continuity, pulse periods, duty cycles, duration of treatment, and/or other different characteristics of emission of radiation. For example, a first type of light therapy may use substantially continuous blue or green light (*e.g.* between about 430 nm and about 550 nm) at an illuminance level appropriate for either open or closed eyes. Such therapy may suppress melatonin, shift the zero-point of the phase of the circadian rhythm, and/or encourage the production of more cortisol. A second type of light therapy may use substantially continuous cyan/green light (*e.g.* between about 500 nm and about 530 nm) at an illuminance level appropriate for either open or closed eyes. Such therapy may suppress melatonin, shift the zero-point of the phase of the circadian rhythm, and/or encourage the production of more cortisol. A third type of light therapy may use substantially continuous red light (*e.g.* between about 590 nm and about 700 nm) at an illuminance level appropriate for either open or closed eyes. Such therapy may not suppress melatonin, nor shift the zero-point of the phase of the circadian rhythm, but encourage the production of more cortisol.

A fourth type of light therapy may use pulsed blue light (*e.g.* between about 460 nm and about 490 nm) at an illuminance level appropriate for either open or closed eyes. Such therapy may only suppress melatonin 1/5 to 1/3 as much as continuous blue/green light, shift the zero-point of the phase of the circadian rhythm, and encourage the production of more cortisol less than continuous blue/green light. A fifth type of light therapy may use pulsed cyan/green light at an illuminance level appropriate for either open or closed eyes. Such therapy may suppress melatonin, shift the zero-point of the phase of the circadian rhythm, and/or encourage the production of more cortisol. A sixth type of light therapy may use pulsed red light at an illuminance level appropriate for either open or closed eyes. Such therapy may not suppress melatonin, nor shift the zero-point of the phase of the circadian rhythm, but encourage the production of more cortisol.

A light therapy regimen may be based on one or more of information related to the size/volume/weight of subject 106, current sleep habits of subject 106, current circadian rhythm of subject 106, information related to the age of subject 106, information related to previously administered light therapy to subject 106, information related to medical parameters pertaining to the status of subject 106, stated and/or provided information from a user 108, subject 106, caregiver, clinician input, guidelines, charts, and/or other information. For example, the recommended light therapy may include about 30 minutes of emission of pulse of blue light, about 1 hour, about 90 minutes, about 2 hours, and/or other periods of active light therapy. The recommended light therapy may be scheduled at different moments or stages of subject's 106 sleeping period. For example, light therapy may be provided 1 hour after the onset of sleep, one hour before the (expected) zero-point of the phase of the circadian rhythm, 1 hour after the (expected) zero-point of the phase of the circadian rhythm, one hour before the (expected) moment of awakening, and/or at other determined times and/or periods. Note that the sample off-set of 1 hour is merely exemplary, and not intended to be limiting in any way.

In some implementations, multiple light therapies as disclosure herein may be combined as a light therapy regimen. In some implementations, the light therapy regimen may be designed to treat Delayed Sleep Phase Syndrome (DSPS) by modifying a phase of the circadian rhythm of the subject. In some implementations, the light therapy regimen may be designed to treat Advanced Sleep Phase Syndrome (ASPS) by modifying a phase of the circadian rhythm of the subject. In some implementations, the light therapy regimen may be designed to treat a teenager who is having trouble falling asleep early enough and/or waking up early enough. In some implementations, the light therapy regimen may be designed to help people transition between different schedules for the weekend and the work week. In some implementations, the light therapy regimen may be designed to help travelers with jet lag. In some implementations, the light therapy regimen may be designed to help people with Monday-morning blues.

For example, treatment of a teenager with DSPS, but unknown (zero-point of the) phase of circadian rhythm, may include the following stages. To start, provide the third type of light therapy (described above) to assist with waking up. Treatment may be augmented by taking extra melatonin before going to sleep and/or wear blue-blocking sunglasses at night. After a number of days, provide the fourth type of light therapy (described above) about 1 hour before the appropriate wake-up time.

For example, a shift worker working the 3^{rd} shift may be treated using the following stages. To start, the second type of light therapy (described above) may be provided throughout the work week during naps before working to shift the zero-point of the phase of the circadian rhythm to be later in the morning. Prior to the work week starting, the second type of light therapy may be provided before bed time until the zero-point of the phase of the circadian rhythm. On the last one or two nights of shift work (par a particular work week), the fourth type of light therapy (described above) may be used in the late morning, when returning home. For the weekend (similar to the previous example) the third type of light therapy (described above) may be used at or near the end of a sleep session to improve performance with increased cortisol, without altering the phase of the circadian rhythm. Combinations, permutations, variations, sequences, and/or alternative scenarios are considered within the scope of this disclosure. Implementations are not intended to be limited to choosing from the six described light therapies above, nor to the combination of merely two types of light therapy in a particular light therapy regimen.

In some implementations, therapy module 112 may be configured to modify a light therapy regimen based on measurements and/or input from a user or care provider, medical professional, and/or doctor. In some implementations, therapy module 112 may automatically and/or autonomously adjust the light therapy regimen based on stated goals and/or targets. For example, adjustments may be made until a patient reaches and/or maintains a particular sleep schedule.

The scheduling of the light therapy may be based on one or more output signals of one or more sensors 142, on one or more parameters determined by parameter determination module 113, on information obtained by/through therapy module 112, on the difference between the expected/measured zero-point of the phase of the circadian rhythm and the desired/recommended zero-point of the phase of the circadian rhythm, and/or any combination thereof. The recommended light therapy regimen may in turn be used as a basis for operations and/or adjustments by light control module 111. Processor 78 may control first lighting module 16 and second lighting module 18 in accordance with the recommended light therapy.

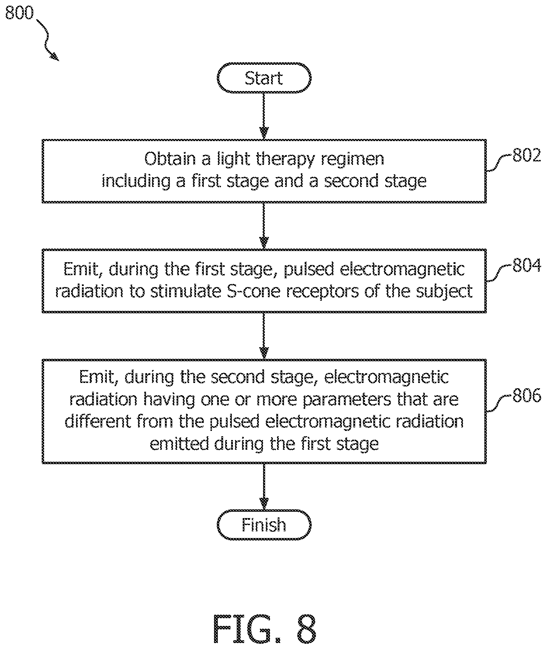

FIG. 8 illustrates a method 800 for providing light therapy to subject 106. The operations of method 800 presented below are intended to be illustrative. In some embodiments, method 800 may be accomplished with one or more additional operations not described, and/or without one or more of the operations discussed. Additionally, the order in which the operations of method 800 are illustrated in FIG. 8 and described below is not intended to be limiting.

In some embodiments, method 800 may be implemented in one or more processing devices (*e.g.,* a digital processor, an analog processor, a digital circuit designed to process information, an analog circuit designed to process information, and/or other mechanisms for electronically processing information). The one or more processing devices may include one or more devices executing some or all of the operations of method 800 in response to instructions stored electronically on an electronic storage medium. The one or more processing devices may include one or more devices configured through hardware, firmware, and/or software to be specifically designed for execution of one or more of the operations of method 800.

At an operation 802, a light therapy regimen is obtained for the subject. The light therapy regimen defines stages including a first stage and a second stage. The first stage is associated with a first period during one or more days. The second stage is associated with a second period during the one or more days that is different from the first period. The light therapy regimen includes electromagnetic radiation impinging on a closed eyelid of the subject to provide light therapy for the subject. In some embodiments, operation 802 is performed by a therapy module the same as or similar to therapy module 112 (shown in FIG. 7 and described herein).

At an operation 804, during the first stage, pulsed electromagnetic radiation is emitted that includes electromagnetic radiation having a first range of wavelengths ranging between about 410 nm and about 510 nm, at a first intensity being sufficient to stimulate S-cone receptors of the subject upon impingement. The pulsed electromagnetic radiation emitted during the first period has a pulse duration up to about 10 minutes and an inter-pulse duration between about 0.1 seconds and about 10 minutes. In some embodiments, operation 804 is performed by a light source and/or a light control module the same as or similar to light source 30 and light control module 111 (shown in FIG. 7 and described herein).

At an operation 806, during the second stage, electromagnetic radiation is emitted wherein one or more parameters of the emitted electromagnetic radiation are changed from the pulsed electromagnetic radiation during the first stage such that light therapy provided during the first stage is different from light therapy provided during the second stage. In some embodiments, operation 806 is performed by a light source and/or a light control module the same as or similar to light source 30 and light control module 111 (shown in FIG. 7 and described herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the embodiments have been described in detail for the purpose of illustration based on what is currently considered to be most practical and preferred, it is to be understood that such detail is solely for that purpose and that the disclosure is not limited to the disclosed embodiments, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A system (10) to provide light therapy to a subject, the system comprising:
one or more light sources (30) configured to emit electromagnetic radiation, wherein the emitted electromagnetic radiation includes electromagnetic radiation having a first intensity and a second intensity, the second intensity being less than the first intensity;
one or more processors (110) configured to execute computer program modules, the computer program modules comprising:
a therapy module (112) configured to obtain a light therapy regimen for the subject, wherein the light therapy regimen defines stages including a first stage and a second stage, wherein the first stage is associated with a first period during one or more days, wherein the second stage is associated with a second period during the one or more days that is different from the first period, wherein the light therapy regimen is configured such that:
during the first stage the subject receives pulsed electromagnetic radiation including electromagnetic radiation having a first range of wavelengths, the electromagnetic radiation during pulses being at a first stage intensity, wherein the pulsed electromagnetic radiation received during the first period has a pulse duration up to about 10 minutes and an inter-pulse duration between about 0.1 seconds and about 10 minutes, and
during the second stage the subject receives electromagnetic radiation, wherein one or more parameters of the electromagnetic radiation during the second stage are changed from the pulsed electromagnetic radiation during the first stage such that light therapy provided during the first stage is different from light therapy provided during the second stage; and
a light control module (111) configured to control emission from the one or more light sources in accordance with the light therapy regimen,
wherein, during the first stage, the first range of wavelengths and the first stage intensity are selected to stimulate S-cone receptors of the subject upon impingement upon a closed eyelid of the subject, **characterized in that** the system, further comprises an eyelid detector, configured and arranged to alter one or more parameters of the electromagnetic radiation in response to a determination that the subject's eyelid is open.

2. The system of claim 1, wherein the pulsed electromagnetic radiation during the first stage is in a predetermined range of wavelengths, wherein the predetermined range of wavelengths ranges between about 410 nm and about 510 nm.

3. The system of claim 1, wherein the received electromagnetic radiation during the second stage has a second range of wavelengths ranging between about 590 nm and 700 nm such that cortisol-production by the subject is stimulated without suppressing melatonin-production.

4. The system of claim 1, wherein the received electromagnetic radiation during the second stage has a second range of wavelengths ranging between about 500 nm and 530 nm such that a phase of the circadian rhythm of the subject is modified.

## Patentansprüche

1. System (10) zum Bereitstellen von Lichttherapie an ein Subjekt, wobei das System umfasst:
eine oder mehr Lichtquellen (30), konfiguriert zum Emittieren elektromagnetischer Strahlung, wobei die emittierte elektromagnetische Strahlung elektromagnetische Strahlung einschließt, die eine erste Intensität und eine zweite Intensität aufweist, wobei die zweite Intensität geringer als die erste Intensität ist;
einen oder mehrere Prozessoren (110), konfiguriert zum Ausführen von Computerprogrammmodulen, wobei die Computerprogrammmodule umfassen:
ein Therapiemodul (112), konfiguriert zum Erhalten eines Lichttherapieregimes für das Subjekt, wobei das Lichttherapieregime Stufen definiert, die eine erste Stufe und eine zweite Stufe einschließen, wobei die erste Stufe einem ersten Zeitraum während eines oder mehrerer Tage zugeordnet ist, wobei die zweite Stufe einem zweiten Zeitraum während des einen oder der mehreren Tage zugeordnet ist, der von dem ersten Zeitraum verschieden ist, wobei das Lichttherapieregime derart konfiguriert ist, dass:
während der ersten Stufe das Subjekt gepulste elektromagnetische Strahlung empfängt, einschließlich elektromagnetischer Strahlung, die einen ersten Bereich von Wellenlängen aufweist, wobei die elektromagnetische Strahlung während der Impulse auf einer ersten Stufenintensität ist, wobei die gepulste elektromagnetische Strahlung, die während des ersten Zeitraums empfangen wird, eine Impulsdauer von bis zu etwa 10 Minuten und eine Interpulsdauer zwischen etwa 0,1 Sekunden und etwa 10 Minuten aufweist, und
während der zweiten Stufe das Subjekt elektromagnetische Strahlung empfängt, wobei einer oder mehrere Parameter der elektromagnetischen Strahlung während der zweiten Stufe gegenüber der gepulsten elektromagnetischen Strahlung während der ersten Stufe derart verändert sind, dass Lichttherapie, die während der ersten Stufe bereitgestellt wird, von der Lichttherapie verschieden ist, die während der zweiten Stufe bereitgestellt wird; und
ein Lichtsteuermodul (111), konfiguriert zum Steuern der Emission von der einen oder den mehreren Lichtquellen gemäß dem Lichttherapieregime, wobei während der ersten Stufe der erste Bereich von Wellenlängen und die erste Stufenintensität ausgewählt werden, um die S-Kegel-Rezeptoren des Subjekts zu stimulieren, wenn diese auf ein geschlossenes Augenlid des Subjekts auftreffen, **dadurch gekennzeichnet, dass** das System weiter einen Augenliddetektor umfasst, der konfiguriert und angeordnet ist, um einen oder mehrere Parameter der elektromagnetischen Strahlung als Reaktion auf eine Bestimmung zu ändern, dass das Augenlid des Subjekts offen ist.

2. System nach Anspruch 1, wobei die gepulste elektromagnetische Strahlung während der ersten Stufe in einem vorbestimmten Bereich von Wellenlängen liegt, wobei der vorbestimmte Bereich von Wellenlängen von etwa 410 nm bis etwa 510 nm reicht.

3. System nach Anspruch 1, wobei die empfangene elektromagnetische Strahlung während der zweiten Stufe einen zweiten Bereich von Wellenlängen aufweist, der von etwa 590 nm und 700 nm reicht, so dass die Cortisolproduktion durch das Subjekt stimuliert wird, ohne die Melatoninproduktion zu unterdrücken.

4. System nach Anspruch 1, wobei die empfangene elektromagnetische Strahlung während der zweiten Stufe einen zweiten Bereich von Wellenlängen aufweist, der von etwa 500 nm und 530 nm reicht, so dass eine Phase des circadianen Rhythmus des Subjekts modifiziert wird.

## Revendications

1. Système (10) pour fournir une luminothérapie à un sujet, le système comprenant :
une ou plusieurs sources de lumière (30) configurées pour émettre un rayonnement électromagnétique, dans lequel le rayonnement électromagnétique émis inclut un rayonnement électromagnétique présentant une première intensité et une seconde intensité, la seconde intensité étant inférieure à la première intensité ;
un ou plusieurs processeurs (110) configurés pour exécuter des modules de programme informatique, les modules de programme informatique comprenant :
un module de thérapie (112) configuré pour obtenir un régime de luminothérapie pour le sujet, dans lequel le régime de luminothérapie définit des stades incluant un premier stade et un second stade, dans lequel le premier stade est associé à une première période au cours d'un ou plusieurs jours, dans lequel le second stade est associé à une seconde période au cours des un ou plusieurs jours qui est différente de la première période, dans lequel le régime de luminothérapie est configuré de sorte que :
au cours du premier stade, le sujet reçoive un rayonnement électromagnétique pulsé incluant un rayonnement électromagnétique présentant une première plage de longueurs d'onde, le rayonnement électromagnétique au cours des impulsions se trouvant à une intensité du premier stade, dans lequel le rayonnement électromagnétique pulsé reçu au cours de la première période a une durée d'impulsion allant jusqu'à environ 10 minutes et une durée entre impulsions entre environ 0,1 seconde et environ 10 minutes, et
au cours du second stade, le sujet reçoive un rayonnement électromagnétique, dans lequel un ou plusieurs paramètres du rayonnement électromagnétique au cours du second stade est ou sont modifiés vis-à-vis du rayonnement électromagnétique pulsé au cours du premier stade de sorte que la luminothérapie fournie au cours du premier stade soit différente de la luminothérapie fournie au cours du second stade ; et
un module de commande de lumière (111) configuré pour commander une émission des une ou plusieurs sources de lumière conformément au régime de luminothérapie, dans lequel, au cours du premier stade, la première plage de longueurs d'onde et l'intensité du premier stade sont sélectionnées pour stimuler des récepteurs de forme conique en S du sujet lors d'une incidence sur une paupière fermée du sujet, **caractérisé en ce que** le système comprend en outre un détecteur de paupière configuré et agencé pour modifier un ou plusieurs paramètres du rayonnement électromagnétique en réponse à une détermination que la paupière du sujet est ouverte.

2. Système selon la revendication 1, dans lequel le rayonnement électromagnétique pulsé au cours du premier stade se situe dans une plage prédéterminée de longueurs d'onde, dans lequel la plage prédéterminée de longueurs d'onde se situe entre environ 410 nm et environ 510 nm.

3. Système selon la revendication 1, dans lequel le rayonnement électromagnétique reçu au cours du second stade a une seconde plage de longueurs d'onde allant entre environ 590 nm et 700 nm de sorte que la production de cortisol par le sujet soit stimulée sans supprimer la production de mélatonine.

4. Système selon la revendication 1, dans lequel le rayonnement électromagnétique reçu au cours du second stade a une seconde plage de longueurs d'onde se situant entre environ 500 nm et 530 nm de sorte qu'une phase du rythme circadien du sujet soit modifiée.
